## Europäisches Patentamt
## European Patent Office
## Office européen des brevets

(19)

(11) Veröffentlichungsnummer: **0 022 973**
**A1**

(12) # EUROPÄISCHE PATENTANMELDUNG

(21) Anmeldenummer: 80103856.3

(22) Anmeldetag: 07.07.80

(51) Int. Cl.³: **C 12 P 17/18**

(30) Priorität: 16.07.79 DD 214335

(43) Veröffentlichungstag der Anmeldung:
28.01.81 Patentblatt 81/4

(84) Benannte Vertragsstaaten:
CH DE FR IT LI

(71) Anmelder: VEB Arzneimittelwerk Dresden
Wilhelm-Pieck-Strasse 35
DDR-8122 Radebeul 1(DD)

(72) Erfinder: Baumert, Alfred, Dr.
Emil-Abderhalden-Strasse 39
DDR-402 Halle(DD)

(72) Erfinder: Erge, Dieter, Dr.
Feuerbachstrasse 73
DDR-402 Halle(DD)

(72) Erfinder: Gröger, Detlef, Prof. Dr.
Hegelstrasse 66
DDR-402 Halle(DD)

(72) Erfinder: Maier, Walter, Dr.
Block 425/7
DDR-409 Halle-Neustadt(DD)

(72) Erfinder: Schmauder, Hans-Peter, Dr. sc.
Brucknerstrasse 16
DDR-402 Halle(DD)

(72) Erfinder: Schumann, Brigitte, Dr.
Leninallee 156
402 Halle(DD)

(74) Vertreter: Beetz, sen., Richard, Dipl.-Ing. Patentanwälte
Dipl.-Ing. R. Beetz sen. Dipl.-Ing. K. Lamprecht;Dr. Ing. R.
Beetz jr. et al,
Rechtsanwalt Dipl.-Phys. Dr. jur. U. Heidrich Dr.-Ing. W.
Timpe; Dipl.-Ing. J. Siegfried Dipl.-Chem. Dr.rer.nat.W.
Schmitt-Fumian Steinsdorfstrasse 10
D-8000 München 22(DE)

(54) Verfahren zur Herstellung von Ergosin.

(57) Um Ergosin breiter pharmazeutischer Verwendung zuzuführen, werden Claviceps-Stämme benötigt, die in der Lage sind, saprophytisch Ergosin zu bilden, das nahezu frei von Nebenalkaloiden ist, und sich durch Stabilität, hohe Alkaloidproduktion und gute Fermentationseigenschaften auszeichnen. Die Aufgabe wird gelöst, indem man zur Herstellung von Ergosin den Stamm Claviceps purpurea (Fr.) Tul. IBP 179, seine Mutanten oder Varianten verwendet. Der neue Stamm bildet ein Gesamtalkaloidgemisch (ca. 900-1300 $\mu$g/ml Nährmedium), von dem mindestens 50 % der Alkaloide mit organischen Lösungsmitteln ausschüttelbar sind (ca. 80-90 % Ergosin/Ergosinin und 10-20 % leicht abtrennbare Substanzen). Der Stamm wird in aerober Fermentation in einem flüssigen Nährmedium, welches eine assimilierbare Kohlenstoffquelle, eine assimilierbare Stickstoffquelle und Mineralsalze enthält, in emerser und/oder submerser Kultur bei einer Temperatur von 20-30°C und einem pH-Wert von 4,0-6,2 während eines Zeitraumes von 7-35 Tagen kultiviert. 95 % der gebildeten Alkaloide werden in das Nährmedium abgegeben, aus dem man Ergosin/Ergosinin isoliert.

EP 0 022 973 A1

Croydon Printing Company Ltd.

## Verfahren zur Herstellung von Ergosin

Die Erfindung betrifft ein Verfahren zur Herstellung
von Ergosin.

Von den über 50 bisher bekannten natürlichen Mutterkornalkaloiden besitzen die Vertreter der Peptidgruppe eine
große Bedeutung in der Medizin, so z. B. Ergotamin,
Ergocornin, Ergokryptin und Ergocristin. Das Ergosin
steht dem Ergotamin sehr nahe und unterscheidet sich
vom letzteren durch Austausch der Aminosäure Phenylalanin
gegen die Aminosäure Leucin in der Cyclolseitenkette.
Ergosin wirkt wie andere Mutterkornalkaloide vom Peptidtyp uterotonisch sowie vasokonstriktorisch und ist deshalb für die Medizin von Bedeutung. Das Ergosin bzw. seine
9,10-Dihydroderivate können als Mittel gegen Migräne bzw.
zur Behandlung von arterieller Hypertonie, peripheren
Gefäßerkrankungen, Störungen der Gehirnzirkulation oder
Herzarrhytmien verwendet werden (siehe z. B. DDR-Patentschrift 128612, DDR-Patentschrift 128613). Auch ist das
Ergosin eine Schlüsselverbindung für Partialsynthesen.

Bisher sind kaum Verfahren bekannt geworden, nach denen saprophytisch Ergosin gewonnen werden kann.
Im britischen Patent 1 401 406 ist beschrieben, daß durch Claviceps purpurea OKI 88/72 neben Ergokryptin, Ergocornin und Ergometrin als Hauptalkaloide (30 %, 28 % bzw. 22 %) auch Ergosin in Mengen von 5 % gebildet wird.

In anderen Patenten, beispielsweise im britischen Patent 1 184 039, wird die gleichzeitige Gewinnung von Ergocornin und Ergosin nebeneinander geschützt. Der geschützte Stamm bildet beide Alkaloide bzw. ihre Isomeren Ergocorninin und Ergosinin in etwa gleichen Mengen bei einem Gesamtalkaloidgehalt von etwa 900 - 1100 µg/ml Nährlösung.

Die mikrobiologische Gewinnung eines von anderen Peptidalkaloiden freien Ergosins ist in der DDR-Patentschrift 129 801 beschrieben. Als Begleitalkaloide treten Clavinalkaloide auf (ca. 10 % der Gesamtalkaloide gegenüber 90 % Ergosin/Ergosinin), die leicht abtrennbar sind.

Die Nachteile der genannten Verfahren bestehen darin, daß entweder eng verwandte Peptidalkaloide nebeneinander vorliegen, die erst auf kompliziertem Wege voneinander getrennt werden müssen, oder daß erst nach langen Fermentationszeiten geringe Ergosinmengen produziert werden.

Es ist das Ziel der Erfindung, die genannten Nachteile der bekannten Verfahren zu überwinden, das Alkaloid-Ergosin nach einem günstigen Verfahren herzustellen und damit seine pharmazeutische Verwendung zu ermöglichen.

Die Erfindung löst die Aufgabe, ein ökonomisch vorteilhaftes Verfahren zur Herstellung von Ergosin zu entwickeln, bei dem die aufwendige Abtrennung verwandter Peptidalkaloide nicht erforderlich ist. Hierzu sind Stämme zu isolieren, die

1. in der Lage sind, saprophytisch Ergosin zu bilden, das nahezu frei ist von Nebenalkaloiden und

2. sich durch Stabilität, hohe Alkaloidproduktion und gute Fermentationseigenschaften auszeichnen.

Erfindungsgemäß werden zur Herstellung von Ergosin der Stamm Claviceps purpurea (Fr.) Tul. IBP 179, seine Mutanten oder Varianten verwendet.

Der neue Stamm Claviceps purpurea (Fr.) Tul. IBP 179 ist beim Zentralinstitut für Mikrobiologie und experimentelle Therapie der Akademie der Wissenschaften der DDR in Jena unter der Bezeichnung IMET PA 136 sowie bei der Deutschen Sammlung von Mikroorganismen (DSM) in 34 Göttingen, BRD am          unter der Nr.          hinterlegt. Er bildet ein Gesamtalkaloidgemisch (ca. 900 – 1300 µg/ml Nährmedium), von dem mindestens 50 % der Alkaloide mit organischen Lösungsmitteln ausschüttelbar sind und davon wiederum mehr als 80 – 90 % durch das Isomerenpaar Ergosin/Ergosinin repräsentiert werden. Die restlichen 10 – 20 % stellen leicht von den Hauptalkaloiden abtrennbare Substanzen (Clavinalkaloide bzw. Ergometrin) dar. Ergokryptin trat teilweise in geringen Spuren auf. In den Nährmedien verbleibt nach dem Ausschütteln u. a. Lysergsäure. Die Alkaloide lassen sich auf bekannten Wegen aus dem Nährmedium isolieren. Eine Umwandlung der isomeren Alkaloide ineinander ist möglich.

- 4 -

Der Stamm zeichnet sich dadurch aus, daß er mehr als 95 % der gebildeten Alkaloide in die umgebenden Nährmedien abgibt und daß er neben Ergosin und dessen Isomeren Ergosinin nur Begleitalkaloide bildet, die leicht abtrennbar sind.

Die Kultivation zur Charakterisierung von Claviceps purpurea (Fr.) Tul. IBP 179, IMET PA 136, erfolgte auf bzw. in den in Tabelle 1 zusammengestellten Medien. Der neue Stamm besitzt die folgenden makroskopischen und mikroskopischen Eigenschaften:

Makroskopisches Aussehen
    Alter der Agarkulturen: 3 Wochen
    Agar NL 126: Flacher, hellbrauner Rasen, feinwollig,
                kaum gefaltet
                Einzelkolonien: ⌀ 0,8 - 1,3 cm, scharf aufgesetzt, hellbraun, feinwollig,
                          im UV-Licht schwache
                          Fluoreszenz

    Agar NL 829: Erhabener, stark gefalteter Rasen, dessen
                Oberseite graubraun ist, oft scharf aufge-
                setzte Ränder.
                Einzelkolonien: ⌀ 0,8 - 1,3 cm, bis 1 cm
                hoch. Napfkuchenförmig aufgesetzt, mit graubrauner Oberseite. Junge Kolonien besitzen
                eine graubraune, alte Kolonien eine mittelbraune Unterseite; Im UV-Licht Fluoreszenz.

Mikroskopisches Aussehen
Vorkultur NL 849:
    7 Tage alt - Das Mycel besteht aus knotenstockartigen
                    Hyphen. Vereinzelt treten längliche,
                    ellipsoide Sporen auf. Hyphen-⌀: 6-8 µm.

0022973

Hauptkultur NL 833:

7 Tage alt - Mycel mit langen, verzweigten Hyphen, deren Wand normal und septiert ist. Die Hyphen wirken "körnig" mit zentralem oder wandständigem Plasma. Blasige Anschwellungen, meist am Hyphenende. Selten Sporen. Hyphen-∅: 5-7 µm.
∅ der blasigen Anschwellungen: 8-10 µm.

14 Tage alt - Das Mycel besteht aus langen, verzweigten, stark septierten Hyphen. Das Plasma ist zentral oder wandständig angeordnet. Hyphen-∅: 5-7 µm.
Knotenstockartige Verdickungen: 8-12 µm, vereinzelt Arthrosporen.

Das Mycel enthält Lipidtröpfchen, deren Anteil in alkaloidbildenden Kulturen besonders hoch ist.

Die Kultivation erfolgte bei 24 $\pm$ 1 $^{\circ}$C. Die Schüttelkulturen wurden auf Rundschütteltischen bei 240 UpM inkubiert.

Die aerobe Fermentation des Pilzes zur Produktion erfolgt zweckmäßigerweise in flüssigen Nährmedien in emerser und/oder submerser Kultur. Die Nährmedien enthalten eine assimilierbare C-Quelle, wie beispielsweise Saccharose, Glucose, Mannit, Sorbit, Glycerin, Zitronen- oder Bernsteinsäure, eine assimilierbare N-Quelle, wie beispielsweise Ammoniak, Asparagin, Pepton, Caseinhydrolysat oder ein Ammoniumsalz, sowie Mineralsalze. Die Fermentation kann beispielsweise in Fernbach-, Erlenmeyer- oder Schüttelkolben oder im Fermenter erfolgen.

Die Kultivation erfolgt zweckmäßigerweise ein- bis dreistufig. Es ist vorteilhaft, wenn die Vorkultur 4-14 Tage alt und das Impfverhältnis Vor-:Hauptkultur = 1:5 bis 1:20 ist. Die Hauptkultur beträgt 7-35 Tage. Der pH-Wert kann zwischen 4,0 und 6,2, die Temperatur zwischen 20 und 30 $^{\circ}$C liegen. Optimal ist eine Fermentation bei 22-25 $^{\circ}$C und pH 5,2 - 5,9.

Die Stammerhaltung kann über Röhrchen, Protoplastenselektion oder durch Tiefkühllagerung unter Verwendung von Schutzkolloiden, wie z. B. 60%ige Saccharoselösung: Magermilch 2:1, auf übliche Weise erfolgen.

Ausführungsbeispiele

Die folgenden Beispiele sollen die Erfindung erläutern. Die verwendeten Nährmedien sind Tabelle 1 zu entnehmen.

Beispiel 1

Ausgehend von Konserven des Stammes Claviceps purpurea (Fr.) Tul.IBP 179 wurden Vorkulturen von 100 ml Medium NL 849 in 500 ml-Schüttelkolben beimpft. Die Kolben wurden 7 Tage bei 24 $\pm$ 1 $^{\circ}$C bei 240 UpM bebrütet. 10 % der erhaltenen Kulturen dienten dazu, 500 ml-Kolben mit je 100 ml Medium NL 720 zu beimpfen. Nach 14-tägiger Bebrütung unter den beschriebenen Bedingungen enthielten die Kulturen 510 µg/ml Alkaloide, die nach bekannten Verfahren aufgearbeitet und isoliert wurden.

Beispiel 2

10 % der in Beispiel 1 genannten Vorkultur wurden zum Beimpfen von 100 ml Nährmedium NL 833 in 500 ml-Kolben benutzt. Nach 14-tägiger Inkubation unter den in Beispiel 1 genannten Bedingungen enthielten die Kolben 946 µg/ml Gesamtalkaloid der folgenden Zusammensetzung: Mit organischen Lösungsmitteln ausschüttelbar: 480 µg/ml, davon 80 % Ergosin/Ergosinin, 15 % Clavinalkaloide und 5 % Ergometrin.

Beispiel 3

Zur Identifizierung der Alkaloide werden 10 ml der Nährmedien alkalisiert und mit Chloroform extrahiert. Der Extrakt wird auf 5 ml eingeengt. Davon werden 2 ml durch präparative Schichtchromatographie an Kieselgel mit Fluoreszenzindikator (Lösungsmittelsystem Chloroform: Äthanol = 8:2) getrennt. Die Alkaloidflecke werden abgeschabt, mit einer Mischung von Wasser:Methanol:Eisessig = 45:45:10 aufgenommen und anschließend mit van Urk's Reagenz zentrifugiert. Der Überstand wird mit einer Quarzlampe 5 min bestrahlt und die Extinktion im Photometer bestimmt. Die Alkaloidmengen können unter gleichen Bedingungen aufgestellten Eichkurven entnommen werden.

Die präparative Gewinnung der Peptidalkaloide wurde auf folgende Weise durchgeführt: 2,5 l Nährlösung wurden alkalisch gestellt (pH 9), erschöpfend mit Chloroform extrahiert und die vereinigten Extrakte im Vakuum eingedampft. Daraus resultieren 1300 mg Rohalkaloidgemisch. Dieses Material wurde in wenig Chloroform gelöst und an einer Kieselgel-Säule chromatographiert. Als Elutionsmittel diente Chloroform, dem steigende Mengen Äthanol (bis 15 %) zugesetzt wurden. Es bildeten sich zwei getrennte, im UV-Licht blau fluoreszierende Zonen. Die rascher wandernde Zone ergab Ergosinin. Nach Umkristallisieren aus Methanol wurden 290 mg Ergosinin ($C_{30}H_{37}O_5N_5$) erhalten, Fp 225 $^oC$ $[\alpha]_D^{29}$ = + 394$^o$ (c = 1 % Chloroform). Hochaufgelöstes Massenspektrum m/e: 547 ($M^+$); Gef. 547, 2807, Ber. 547, 2794 für $C_{30}H_{37}O_5N_5$. Weitere prominente Fragmente m/e: 280; 267; 224; 221; 210; 167; 154.

Das Eluat der langsamer wandernden Zone enthielt 680 mg Ergosin ($C_{30}H_{37}O_5N_5$). Massensprektrum m/e: 547 ($M^+$). Weitere prominente Fragmente m/e: 280; 267; 223; 221; 210; 167; 154.

In beiden Fällen war die Keller'sche und van Urk'sche Reaktion positiv, und das Absorptionsspektrum im UV zeigte bei $\lambda$ = 312 nm ein Maximum.

Bei der sauren Hydrolyse entstanden jeweils Leucin und Prolin. Die alkalische Hydrolyse lieferte Lysergsäure (Ergosin) bzw. Isolysergsäure (Ergosinin).

Auch das chromatographische Verhalten der beiden isolierten Peptidalkaloide stimmte mit authentischem Ergosin bzw. Ergosinin überein. Eine Umwandlung der Alkaloide ineinander nach bekannten Methoden war möglich.

Tabelle 1

Zusammensetzung der verwendeten Nährlösungen
(Angaben in g/l).

| | NL 849 | NL 720 | NL 833 | NL 126 | NL 829 |
|---|---|---|---|---|---|
| Saccharose | 300,0 | 200,0 | 300,0 | - | 100,0 |
| Magermilch | 100 ml | - | - | - | - |
| Bierwürze | - | - | - | 150,0ml | - |
| Ammoniumcitrat | 10,0 | 15,0 | 20,0 | 1,0 | - |
| Asparagin | - | - | - | - | 10,0 |
| Hefeextrakt | - | - | - | 5,0 | 0,5 |
| $Ca(NO_3)_2$ | - | 1,0 | 1,0 | - | 1,0 |
| $KH_2PO_4$ | 0,25 | 0,25 | 0,25 | - | 0,25 |
| $(NH_4)_2HPO_4$ | - | - | - | 5,0 | - |
| $MgSO_4 \cdot 7H_2O$ | 0,3 | 0,3 | 0,3 | - | 0,25 |
| $FeSO_4 \cdot 7H_2O$ | - | 0,010 | 0,010 | - | 0,020 |
| $ZnSO_4 \cdot 7H_2O$ | - | 0,0308 | 0,0308 | - | 0,02 |
| KCl | - | 0,1 | - | - | - |
| Aqua dest.ad | 1000,0 | 1000,0 | 1000,0 | - | 1000,0 |
| Agar-Agar | - | - | - | 30,0 | 30,0 |
| pH vor dem Sterilisieren | 5,4 | 5,8-6,0 | 5,4 | 6,0 | 5,4 |
| pH Einstellung mit | NaOH | NaOH | NaOH | NaOH | NaOH |
| Autoklavieren | Jeweils einheitlich 30 min 110 °C | | | | |

Patentansprüche

1. Verfahren zur Herstellung von Ergosin, dadurch gekennzeichnet, daß der Stamm Claviceps purpurea (Fr.) Tul. IBP 179, IMET PA 136, seine Mutanten oder Varianten verwendet werden.

2. Verfahren nach Anspruch 1, dadurch gekennzeichnet, daß der Stamm Claviceps purpurea (Fr.) Tul. IBP 179, IMET PA 136, in aerober Fermentation in einem flüssigen Nährmedium, welches eine assimilierbare Kohlenstoffquelle, eine assimilierbare Stickstoffquelle und Mineralsalze enthält, in emerser und/oder submerser Kultur bei einer Temperatur von 20 - 30 °C und einem pH-Wert von 4,0 - 6,2 während eines Zeitraumes von 7 - 35 Tagen kultiviert und das gebildete Ergosin/ Ergosinin aus dem Nährmedium auf an sich bekannte Weise isoliert wird.

3. Verfahren nach den Ansprüchen 1 und 2, dadurch gekennzeichnet, daß die Fermentation bei 22 - 25 °C und pH 5,2 - 5,9 durchgeführt wird.

0022973

Europäisches
Patentamt

EUROPÄISCHER RECHERCHENBERICHT

Nummer der Anmeldung
EP 80 10 3856

| | EINSCHLÄGIGE DOKUMENTE | | KLASSIFIKATION DER ANMELDUNG (Int Cl ³) |
|---|---|---|---|
| Kategorie | Kennzeichnung des Dokuments mit Angabe, soweit erforderlich, der maßgeblichen Teile | betrifft Anspruch | C 12 P 17/18 |
| D | DE - A - 2 801 453 (AKADEMIE DER WISSENSCHAFTEN DER DDR)  * Das ganze Dokument *  & DD - A - 129 801  -- | 1-3 | |
| | CHEMICAL ABSTRACTS, Band 75. Nr. 4, 26. Juli 1971, Zusammenfassung Nr. 25298q, Seite 313, Columbus, Ohio, US, A.N. BAN'KOVSKAYA et al.: "Level and qualitative composition of alkaloids from ergot, a parasite on wild grasses and hybrid plants"  & Tr. Vses. Nauch. Issled. Inst. Lek. Aromat. Rast., 1969, 15, 442-56  * Zusammenfassung *  -- | 1 | RECHERCHIERTE SACHGEBIETE (Int. Cl.³)  C 12 P 17/18 |
| A | CHEMICAL ABSTRACTS, Band 71, Nr. 11, 24. November 1969, Zusammenfassung Nr. 100369m, Seite 208, Columbus, Ohio, US, A.M. AMICI et al.: "Production of peptide ergot alkaloids in submerged culture by three isolates of Claviceps purpurea"  & Appl. Microbiol., 1969, 18(3), 464-8  ---- | | KATEGORIE DER GENANNTEN DOKUMENTE  X: von besonderer Bedeutung  A: technologischer Hintergrund  O: nichtschriftliche Offenbarung  P: Zwischenliteratur  T: der Erfindung zugrunde liegende Theorien oder Grundsätze  E: kollidierende Anmeldung  D: in der Anmeldung angeführtes Dokument  L: aus andern Gründen angeführtes Dokument  &: Mitglied der gleichen Patentfamilie, übereinstimmendes Dokument |
| | Der vorliegende Recherchenbericht wurde für alle Patentansprüche erstellt. | | |
| Recherchenort Den Haag | Abschlußdatum der Recherche 31-10-1980 | Prüfer DESCAMPS | |